# EUROPEAN PATENT SPECIFICATION

(11) **EP 3 714 908 B1**
(45) Date of publication and mention of the grant of the patent: **06.03.2024**
(21) Application number: 20166199.8
(22) Date of filing: 27.03.2020
(51) Int. Cl.: A61L 15/42, A61L 15/46, A61L 15/62, A61F 13/00

(54) **A DEVICE FOR WOUND CARE, METHOD TO MANUFACTURE AND USES THEREOF**
WUNDPFLEGEVORRICHTUNG, VERFAHREN ZU IHRER HERSTELLUNG UND IHRE VERWENDUNGEN
DISPOSITIF POUR LE TRAITEMENT DES PLAIES, SON PROCÉDÉ DE FABRICATION ET SES UTILISATIONS

(30) Priority: 29.03.2019 US 201962826511 P
(43) Date of publication of application: 30.09.2020
(73) Proprietor: Picosun Oy, 02150 Espoo (FI)
(72) Inventor: Ritasalo, Riina, 02760 Espoo (FI); Oksala, Niku, 33700 Tampere (FI)
(74) Representative: Heinonen & Co

(56) References cited:
- WO-A2-2010/052190
- US-A1- 2005 221 072
- US-A1- 2007 203 442
- POORIA MOSTAFALU ET AL: "Smart Bandage for Monitoring and Treatment of Chronic Wounds", SMALL, vol. 14, no. 33, 1 August 2018 (2018-08-01), page 1703509, XP055719474, ISSN: 1613-6810, DOI: 10.1002/smll.201703509
- NARAYAN R J ET AL: "Atomic Layer Depoition of TiO2 Thin Films on Nanoporous Alumina Templates: Medical Applications", JOM: JOURNAL OF METALS, SPRINGER NEW YORK LLC, UNITED STATES, vol. 61, no. 6, 1 June 2009 (2009-06-01), pages 12-16, XP001546106, ISSN: 1047-4838

## Description

### FIELD OF THE INVENTION

The present invention generally relates to devices for controlled drug release. More specifically, the invention pertains to a device for wound care comprising a coating film configured for gradual release of wound healing compounds into skin in highly controlled manner.

### BACKGROUND OF THE INVENTION

To protect wounds on skin, related wound care and/or wound dressing products are utilized to facilitate the healing process. Commonly, wound care products create a physical barrier between the wound and the external environment. In some cases, wound treatment may require topically applied medications that further promote healing, prevent inflammation and/or alleviate pain.

Wound care products are typically tailored to suit the wound type and the phase of wound repair. Therefore, for example, treatment of a clean surgical wound, a contaminated surgical wound, dry or moist wound or combinations of these, requires a specific wound care product.

Currently available wound care products include wound tapes, bandages, patches, wound dressings, and plasters, which are known to a person skilled in the art. In addition, numerous available wound care materials including mixtures of vapor-permeable adhesive films, collagen, hydrogels, foams, hydrocolloids, alginates, growth factors, silver impregnated materials, silicone meshes, tissue adhesives, tulles, barrier films, antibiotics, agents promoting debridement of wounds and formation of granulation tissue, are regularly used in patient care to promote wound healing process.

Wound dressings are generally classified as dry dressings, moisture-keeping dressings, bioactive dressings and, ultimately, skin substitutes. Desired properties include provision of mechanical protection, pain relief, absorption of exudate from the wound and, at the same time, ability to maintain or regulate the moisture level, thus facilitating a repair process of the wound. Wounds characterized by marked exudation benefit from absorptive materials that dry the wound, whereas dry wounds benefit from maintenance of the adequate moisture level. Depending on the phase of wound repair, its exposure to oxygen and humidity may also need to be controlled. Hence, the desired properties include controlled exchange of gas and/or fluids. Moreover, wound care products can be used in combination with wound care materials and other topical medication to facilitate wound repair, according to principles known to a skilled person, and are described in the prior art. For example, publication WO 2010/052191 discloses coated solid pliant materials with the coatings providing photocatalytic, antimicrobial and/or immunomodulatory properties to the material. Publication WO 2010/052190 further discloses wound dressings, such as textiles and fabrics, provided with a homogenous and substantially amorphous layer of a metal oxide comprising predominantly titanium oxide.

A major limitation of available solutions is insufficient selection of the active drug delivery systems capable for controlled drug release over an extended period of time. Known systems lack capacity for controlled release of a specific drug or a combination of drugs in constant or dynamically modified rate or release of different drugs such as e.g. local antiseptics, antibiotics, local pain medications, wound repair promoting agents etc., during predetermined phases of wound repair.

In this regard, an update in the field of developing devices for controlled drug release is still desired, in particular, in view of addressing challenges associated with wound care products.

### SUMMARY OF THE INVENTION

The scope of the invention is defined by the claims. Any references in the description to methods of treatment refer to the products of the present invention for use in a method for treatment.

An objective of the present invention is to solve or to at least alleviate each of the problems arising from the limitations and disadvantages of the related art. The objective is achieved by various embodiments of a device for wound care, a method of its manufacturing and of a device for use in a method of wound care. In an aspect, a device for wound care is provided according to what is defined in independent claim 1.

In embodiment, the device is configured as any one of: a bandage, a patch, a tape, a roll, a plaster, and a wound dressing.

In embodiment, the coating film comprises at least one compound selected from aluminium oxide, titanium oxide, silicon oxide, and a combination thereof.

In embodiment, the functional layer comprises at least one chemical substance with antimicrobial- and/or antibacterial activity.

In embodiment, the device further comprises electrical means, such as at least one sensor and related circuitry, for detecting, from skin, at least one predetermined condition selected from temperature, moisture, ionic content and/or dissolution rate of chemical substance(s) released from the functional layer, for example.

In an aspect, a method of manufacturing a device for wound care is provided according to what is defined in independent claim 8.

In embodiment, the method comprises depositing the coating film, which comprises at least one metal oxide. In embodiment, the method comprises depositing the coating film, which comprises at least one compound selected from aluminium oxide, titanium oxide, silicon oxide, or a combination thereof.

In embodiment, the method further comprises a step of arranging, within the device, at least one sensor and related circuitry for detecting, from skin, of at least one predetermined condition selected from temperature, moisture, ionic content and/or dissolution rate of chemical substance(s) released from the functional layer, for example.

In an aspect, the device according to the invention is for use in a method of wound care according to what is defined in independent claim 12.

In embodiment, said device is provided for controlling release of at least one chemical substance into skin, said substance being a biologically and/or pharmaceutically active compound. In embodiment, said device is provided for detecting at least one predetermined condition from skin selected from temperature, moisture, ionic content and/or dissolution rate of chemical substances. Overall, the device 100 can be configured as a surgical tape, bandage, patch and/or different types of dressings (dry, moisture-keeping, bioactive) and skin substitutes aiming at enabling safe and rapid wound healing in comfortable and cost-effective manner.

Without limiting the scope and interpretation of the patent claims, certain technical effects of one or more of the example embodiments disclosed herein are listed in the following.

The presence of coating layer(s) comprising predetermined compounds, such as aluminum oxides, for example, in wound care devices may generate the possibility to reactivate the antimicrobial, anti-fouling, antiviral and/or immunomodulatory activities of the wound care by simple photo-activation. This has an advantage of enabling prolonged local wound care without replacing the dressing every second day, as it is the general rule today. Therefore, the wound is undisturbed and the healing is more efficient.

Furthermore, disclosed technology makes use of free radicals, released from the metal oxides after photo-activation, to eliminate harmful microorganisms and to relieve inflammation. This obviates or at least reduces the need in antibiotic treatments and reduces risks for development of antibiotic-resistant infections. The oxide compounds disclosed hereby are promising materials for developing next generation of bioactive wound care products.

The invention allows for manufacturing smart-body on-skin patches for home- and hospital healthcare for any kind of wounds and burns.

The device according to the invention allows for controlled medicine release for open wounds, burns or similar injuries through the skin-contact surface(s). The device provided hereby enables prolonged and/or gradual medicine release (time-wise) along with increased sterility management due to antimicrobial- or antibacterial layer solutions, for example. The invention further allows for detecting and measuring at least one condition related to wound / skin.

The wound care device disclosed hereby combines a variety of biological functionalities (antibacterial, antimicrobial, etc.) with enabling a controlled release of medicine through the skin/wound contact. The device utilizes biocompatible and safe materials. The invention offers a modern wound dressing solution with embedded drugs/medicine that play an active role in the wound healing process either directly or indirectly as a cleansing agent, for example.

In the present disclosure, materials with a layer thickness below 1 micrometer (µm) are referred to as "thin films".

The expression "a number of" refers herein to any positive integer starting from one (1), e.g. to one, two, or three; whereas the expression "a plurality of" refers herein to any positive integer starting from two (2), e.g. to two, three, or four.

The terms "first" and "second" are not intended to denote any order, quantity, or importance, but rather are used to merely distinguish one element from another.

### BRIEF DESCRIPTION OF THE DRAWINGS

The invention will now be described, by way of example only, with reference to the accompanying drawings, in which:
Fig. 1 schematically illustrates a device 100 according to an embodiment;
Figs. 2A and 2B schematically illustrate a top perspective view and a side view, accordingly, of the device 100, according to some embodiment.

### DETAILED DESCRIPTION

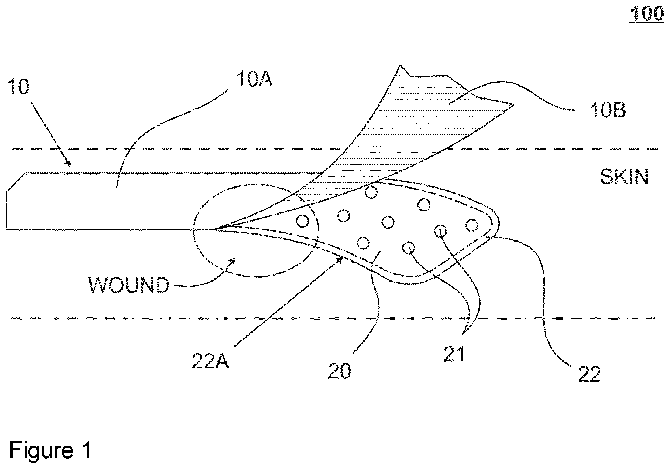

Fig. 1 illustrates, at 100, a concept underlying a device for wound care, hereafter, a device, according to various embodiments.

The device 100 comprises a base layer 10 and a functional layer 20 supported on said base layer and containing at least one chemical substance 21 suitable for wound care. To avoid confusion, we emphasize that the base layer 10 serves as a base (support) layer upon assembling the device 100. When the device is in use (brought into contact and optionally attached) to skin, the base layer 10 becomes a topmost layer. The base layer thus comprises an outer surface 10A and an inner surface 10B. The outer surface 10A is a surface that faces and/or encounters the external environment surrounding the skin and/or wound area, and the inner surface 10B is a surface onto which the functional layer 20 is applied to.

The base layer 10 is provided as essentially flexible (deformable), elastic material. Alternatively, the base layer can be provided as a solid non-pliant material. A support material forming the base layer is advantageously porous thus allowing air penetrating therethrough. The base layer may further comprise a protective layer, e.g. a water resistant layer (not shown), on its surface opposite to the surface onto which the functional layer 20 is supported.

The functional layer 20 comprises the substance 21 optionally carried in a suitable carrier material. The substance 21 is advantageously a compound or compounds that possess(es) biological and/or pharmaceutical activity. The substance 21 can be configured as any one of the: antibacterial, antimicrobial, antibiotic, antiviral, analgesic, anesthetic, growth factor or any other therapeutic substance, or combination of those.

It is preferred that the functional layer comprises at least one chemical substance with antimicrobial- and/or antibacterial activity. By a substance with antibacterial activity we generally refer, in the present disclosure, to a substance that prevents the development of bacteria on a treated surface. Accordingly, by a substance with antimicrobial activity we generally refer to a substance that prevents spread and development of bacteria, fungi and viruses on a treated surface.

The device further comprises a coating film 22 laid over said functional layer 20, whereby the functional layer 20 becomes enclosed between the base layer 10 and the coating film 22. The coating film has a skin-contact surface 22A.

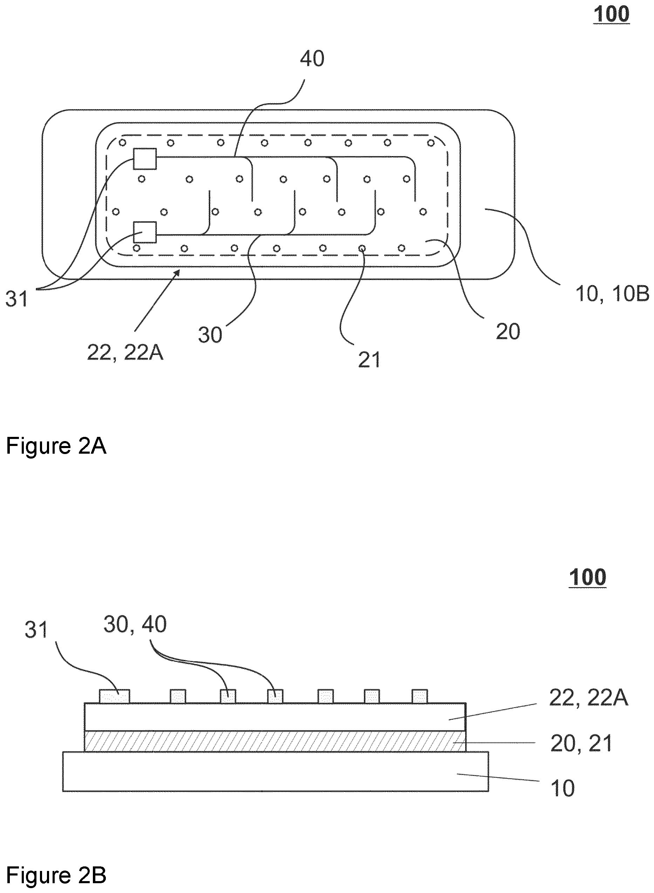

In configuration of Fig. 1, the device 1 is an essentially flat, planar item of finite size applicable onto skin and/or on/around a wound (place of the latter is outlined on Fig. 1 in a circle). Fig. 1 thus shows an exemplary configuration of the device 100 embodied as band plaster (also referred to as a bandage or a first-aid plaster). In such configuration, the inner surface 10B of the base layer 10 may have prominent edges projecting over the area occupied by the functional layer 20 and the coating film 22 (see Fig. 2). This edge area may be supplied with adhesive to render the base layer attachable to skin. In such an event, the wound is at least partially covered with the functional layer 20. The device 100 configured as a bandage can be provided in various size and shapes, as any conventional item of that kind.

The device can be configured as an item for single use (separately packed) or as a roll (plaster tape roll), which can be cut into pieces when needed.

Various configurations include, but are not limited to a bandage, a patch, a tape, a roll, a plaster, and a wound dressing. In some instances, the device is configured as a bandage / a tape without an adhesive.

The device 100 can be further configured as a mesh-type breathable plaster / bandage or a semi-permeable plaster. In some instances, the device 100 is configured as a mesh type implant / a surgical mesh.

It is generally preferred that the base material 10 and/or any one of the layers 20, 22 do not adhere to the wound; therefore, the device 100 is easily removable / replaceable. In some configurations, the base layer material is selected such as to remain attached to surrounding healthy skin to increase the patient's comfort and to prevent the wound from contamination from external sources. The functional layer 20 and optionally the coating film 22 is/are configured, in turn, to promote wound repair by accelerating re-epithelialization, controlling- or inhibiting infection and promoting removal of wound debris. The materials 10, 20, 22 are preferably selected such, as to prevent the ingrowth of repair tissue thereinto. To avoid wound irritation, the materials 10, 20, 22 are non-toxic, non-sensitizing and non-allergenic.

The coating film 22 is established over the functional layer 20 and optionally over at least a part of the base layer 10 using a method of chemical deposition in gaseous (vapour) phase, such as Atomic Layer Deposition (ALD) or, alternatively, Chemical Vapour Deposition (CVD).

The basics of an ALD growth mechanism are known to a skilled person. ALD is a special chemical deposition method based on the sequential introduction of at least two reactive precursor species to at least one substrate. It is to be understood, however, that one of these reactive precursors can be substituted by energy when using, for example, photon-enhanced ALD or plasma-assisted ALD, for example PEALD, leading to single precursor ALD processes. For example, deposition of a pure element, such as metal, requires only one precursor. Binary compounds, such as oxides can be created with one precursor chemical when the precursor chemical contains both of the elements of the binary material to be deposited. Thin films grown by ALD are dense, pinhole free and have uniform thickness.

The at least one substrate is typically exposed to temporally separated precursor pulses in a reaction vessel to deposit material on the substrate surfaces by sequential self-saturating surface reactions. In the context of this application, the term ALD comprises all applicable ALD based techniques and any equivalent or closely related technologies, such as, for example the following ALD sub-types: MLD (Molecular Layer Deposition), plasma-assisted ALD, for example PEALD (Plasma Enhanced Atomic Layer Deposition) and photon-enhanced Atomic Layer Deposition (known also as photo-ALD or flash enhanced ALD). The process can also be an etching process, one example of which being an ALE process. It should be noted that with PEALD and photon-enhanced ALD, the additive treatment can be limited to the surfaces visible to the radiation source.

ALD is based on alternating self-saturative surface reactions, wherein different reactants (precursors) provided as chemical compounds or elements in a nonreactive (inert) gaseous carrier are sequentially pulsed into a reaction space accommodating a substrate. Deposition of a reactant is followed by purging the substrate by inert gas. Conventional ALD deposition cycle proceeds in two half-reactions (pulse A - purge A; pulse B - purge B), whereby a layer of material is formed in a self-limiting (self-saturating) manner, typically being 0,05 - 0,2 nm thick. Typical substrate exposure time for each precursor ranges within 0,01 - 1 seconds per chemical, each pulse being alternated with an about 10 s purge. In order to deposit a layer with thickness 10 - 100 nm, the deposition procedure may be completed within a time range of about 10 minutes to about 20 hours, depending on a number of cycles required and duration of each cycle. In similar manner, the coating film can be established from three or more precursors.

Pulse A comprises a first precursor in a gaseous phase (first precursor vapor) and pulse B comprises a second precursor in a gaseous phase (second precursor vapor). Inactive gas and a vacuum pump are typically used for purging gaseous reaction by-products and the residual reactant molecules from the reaction space during purge A and purge B. A deposition sequence comprises at least one deposition cycle. Deposition cycles are repeated until the deposition sequence has produced a thin film or coating of desired thickness. Deposition cycles can also be either simpler or more complex. For example, the cycles can include three or more reactant vapor pulses separated by purging steps, or certain purge steps can be omitted. On the other hand, photo-enhanced ALD has a variety of options, such as only one active precursor, with various options for purging. All these deposition cycles form a timed deposition sequence that is controlled by a logic unit or a microprocessor.

The coating film 22 is formed by depositing at least one coating layer onto an underlying substrate. The coating film 22 can be configured as a nanolaminate established on a support material using a chemical deposition method. Deposition of the exemplary coating film 22 exploits the principles of atomic layer deposition (ALD) and provides for sequential, temporally separated delivery of at least two different precursors into the reaction space. Precursors are preferably distinct chemicals selected such, as to form a preselected compound or composition 22 (hereby, the coating) on a surface of the substrate.

Selected ALD processes to deposit the film 22 are preferably conducted at low temperatures. The ALD reaction(s) is/are performed at a reaction temperature within a range of about 20-500 degrees centigrade (°C), such as between about 20-400 °C, about 20-300 °C, about 20-200 °C, about 20-100 °C, about 50-300 °C, about 50-200 °C, about 50-150 °C, or about 100-200 °C. In some preferred configurations, the deposition reaction is conducted within a range of about 100-200 °C, preferably, at about 120 °C.

Deposition thickness of the coating film 22 is within a range of about 0,03 nm to about 100 nm.

By virtue of being coated by the ALD film, the device 100 can be rendered with functionality of controlled release and delivery of wound healing compounds (in)to skin and/or the wound. The coating film 22 is thus configured, upon being brought into contact with skin via its skin-contact surface 22A, to dissolve and to release the chemical substance 21 to skin through the skin-contact surface 22A.

The coating film 22 is configured for gradual, time-controlled dissolution. This enables adjustment (increase or decrease) of a chemical substance release gradient over a dissolving time of chemically deposited film coating.

The coating layer 22 contains at least one metal-containing compound, such as metal oxide. The metal oxide compound can be selected from aluminium oxide (Al₂O₃), titanium oxide (TiO₂) and combination thereof. In some instances, the coating layer contains a non-metal compound, such as silicon oxide (SiO₂) or a combination of silicon oxide with aluminium oxide and/or titanium oxide. Aluminium oxide is particularly beneficial for establishing a controlled release of chemical substances into skin. Al₂O₃ dissolves in a body-fluid-environment, including that created upon contact with a (saline-containing) wound environment.

The coating film 22 configured as (nano)laminate containing material layers deposited with Al₂O₃, SiO₂ and/or Al₂O₃-SiO₂ causes, upon dissolution, release of the chemical substance 21 from the functional layer 20 to skin (the skin area surrounded by the edges / borders of base layer 10) in a highly controlled manner. Release of the chemical substance 21 can be controlled by varying thickness (viz. a number of material layers) in said (nano)laminate.

Additionally, the coating film 22 provided as the (nano)laminate produced from the above mentioned compounds can be configured bendable (i.e. capable to flex together with flexible support / base layer 10).

ALD-deposited aluminium oxide and/or silicon oxide provide for well-controlled medicine release. Selected ALD material(s), such as Al₂O₃ or SiO₂, for example, are biocompatible and dissolve, when brought into contact with biological (bodily) fluids. The amounts of said compounds utilized in the ALD process are extremely small (due to the fact that the nanometer scale layers are produced). Additionally, some compounds, such as Al₂O₃ film, for example, can be ALD-deposited at essentially low-temperature, within a range of about 20 °C to about 100 °C.

ALD is a conventionally conformal process that allows for controlling layer material thicknesses at angstrom level. Abovementioned Al- and Si oxides dissolve in a controlled manner when brought into contact with bodily fluids. By controlling thickness of the coating film 22 deposited from Al₂O₃ and/or SiO₂, a dissolution time of said film and subsequent medicine 21 release can be regulated with high precision. Release time may be extended by embedding the medicine 21 into the ALD layer (wherein the medicine becomes distributed in the ALD layer, through the depth of the coating film 22). This enables establishing a prolonged healing effect through the open wounds or burns.

A multilayer structure can be constructed comprising a stack (not shown), in which a number of the functional layers 20 alternate by means of a corresponding number of coating films 22 (the latter acting as "separating membranes"). The entire stack is placed onto the support / base layer 10 and coated with a topmost coating film thus having the skin-contact surface 22A. The device may thus comprise more than one functional layer 20; each such functional layer may be provided with same- or different chemical substance. By way of example, a stack structure forming the device 100 can be provided, comprising: a base layer 10 + 1^{st} functional layer 20-1 (containing 1^{st} chemical substance 21-1 / "drug 1") + 1^{st} coating film 22-1 (Al₂O₃) + 2^{nd} functional layer 20-2 (containing 2^{nd} chemical substance 21-2 / "drug 2") + 2^{nd} coating film 22-2 (SiO₂) + 3^{rd} functional layer 20-3 (containing 3^{rd} chemical substance 21-3 / "drug 3") + 3^{rd} coating film 22-3 (Al₂O₃). In present example, the coating film 22-3 has a skin-contact surface 22A.

In some configurations, the functional layer 20 and the coating film 22 laid over it may be provided at both faces of the base layer 10 (not shown).

A functional area is created by the at least one functional layer 20. The device 100 may further comprise more than one said functional area on the same base substrate 10. Functional areas 20 may be disposed adjacent to one another or arranged into arrays. The coating film 22 may be provided to coat each said functional area 20 separately or, alternatively, all functional areas 20 may be coated with the same coating film 22. Each said functional area 20 may include the same chemical substance 21 or different chemical substances. The device 100 may thus be configured as a kit-of-parts comprising a number of "patches" with different functionality that can be separated from one another by e.g. cutting the substrate area 10 between the functional areas 20 (not shown).

Additionally or alternatively, the metal oxide can be provided as zinc oxide (ZnO). In some embodiments, the metal oxide layer comprises a metal selected from the group consisting of palladium, platinum, ruthenium, iridium, osmium and rhodium.

In some instances, the coating layer can comprise metal carbide or metal nitride. In some further instances, the coating layer can comprise metal oxycarbide, such as aluminium oxycarbide, or oxynitride, such as titanium oxynitride.

The ALD film 22 is thus provided as a coating for the separate medicine layer 20 (Fig. 1, Fig. 2B).

In some instances, the ALD layer can be provided as a medicine layer. Such configuration may be illustrated by Fig. 2A. Hereby, the coating film 22 may be implemented as a functional layer 20. In such an event, the substance 21 may embedded into the coating film 22 and distributed through the depth of said film. Herein, the (ALD) coating layer acts as a carrier for the at least one chemical substance 21 optionally configured as a pharmaceutically active compound.

Still, the configuration of Fig. 2A is also applicable to a situation, when the separate functional layer 20 (shown in a dashed line) is disposed between the base substrate 10 and the coating film 22.

Figs. 2A and 2B show the device 100 further comprising means 30, 40, such as electrical means configured as at least one sensor / detector to detect at least one condition from skin including, but not limited to temperature, moisture, ionic content and/or dissolution rate of chemical substance(s). Said at least one sensor 30, 40 is supplied with related (electric) circuitry (not shown). In some instances, a number of sensors 30, 40 is provided to measure different conditions from skin. The detectors / sensors and/or the sensing circuitry can be placed on any surface of the device 100, such as on/within the functional layer / the skin-contact surface 22A (e.g. the antimicrobial surface, a pharmacologically/biologically active side facing the wound) or on the external surface 10A or the internal surface 10A of the device. Said electrical means 30, 40 are connected to contact pads 31, e.g. electrode pads.

In exemplary configuration, the electrical means 30, 40 are configured is interdigitated electrodes (IDE).

Signals produced by said means 30, 40 may be transmitted and/or read by external devices (e.g. wireless transmission commonly used in RFID's). The electrical means may be powered externally.

Provision of sensing means 30, 40 and related sensing circuitry for detection of a predetermined condition or conditions has the following benefits. In severe cases (e.g. burns), change of a bandage may require extreme care and special hygienic conditions. Tearing of adhesive surfaces from skin, in particular, in infants, may cause damages and prolong wound healing. By using sensors 30, 40 and external measurement equipment that receives signals from said sensors, a condition / state of skin and the wound can be evaluated without the need to remove the bandage 100.

The invention further pertains to provision of a method for manufacturing a wound care device 100. The method comprises obtaining the base layer 10 as described hereinabove and applying the functional layer 20 containing at least one chemical substance 21 suitable for wound care onto said base layer. The functional layer 20 can be applied by any conventional method, such as printing, dispensing or patterning. Alternatively, the functional layer 20 can be applied by any method of chemical deposition in vapour phase described hereinabove.

The method continues at depositing, by atomic layer deposition (ALD), the coating film 22 over said functional layer 20 such, that upon bringing said coating film into contact with skin via the skin-contact surface 22A, the coating film dissolves and releases the chemical substance(s) 21 to skin through the skin-contact surface.

In some instances, the coating layer comprises an oxide layer, such as aluminium oxide, titanium oxide, silicon oxide, or a combination thereof.

ALD technology is utilized hereby to realize the controlled release of desired chemical substances, such as wound-healing drugs, in order to control infections and to promote wound repair. Material selection and thickness of the ALD coating film 22 is/are preferably regulated such as to generate microsized pores in said film. By careful selection of deposition materials and/or reaction conditions, the (micro)pore size can be adjusted and therefore gas-fluid exchange rate can be modulated to maintain the wound homeostasis.

By proper material selection and/or regulating the ALD deposition cycle conditions, pore size can be influenced such, that when combined with the controlled dissolution rate, it can result in formation of a product with improved breathing properties. Thus, each material layer within the (nano)laminate (viz. the film 22) described hereinabove can be rendered with different pore size. For example, the material layers with greater pores can be arranged closer to skin (at the skin-contacting surface 22A). Materials with greater pore size disintegrate more rapidly, which can be used in regulating dissolution rate of chemical substances / drugs provided in the functional layer 20.

While the pores, such as (micro)pores, are mainly needed to provide wound homeostasis environment, the drug release is mainly controlled by the thickness of coating material layers. The coating film 22 deposited from predetermined materials (e.g. Al₂O₃) dissolves when brought into contact with biological (bodily) fluids and/or tissues. By modulating the thickness of said coating film, dissolving time can be controlled with high precision. Thin Al₂O₃ layer (about 10 nm) dissolves faster and releases a drug much more promptly than a thicker Al₂O₃ layer (about 100 nm), which naturally needs more time to dissolve. Controlled drug release can be implemented with a combination of Al₂O₃ with any one of SiO₂ or TiO₂. Also partial ALD coating, implemented via essentially non-conformal ALD coating (e.g. PEALD coating) or a patterned coating, for example, can be used to control drug release.

Via time-controlled drug release, the desired healing effect can be promoted / accelerated or otherwise regulated.

ALD is utilized to maximize biocompatibility by creating a non-toxic, non-sensitizing and non-toxic interface between the wound and the ambient / external conditions. By controlling the layer thickness and the size of (micro)pores, the effective time of medicine release and wound homeostasis can be adjusted.

The method further comprises a step of arranging of at least one sensor 30, 40 and related circuitry for detecting, from skin, of at least one predetermined condition selected from temperature, moisture, ionic content and/or dissolution rate of chemical substance(s) / drugs 21 released from the functional layer 20. Said sensor(s) can be arranged within the device 100 before or after the ALD coating based on intended location of said sensors and related circuitry in the device.

The device 100 can be advantageously used in controlling release of at least one chemical substance into skin, said substance configured as a biologically and/or pharmaceutically active compound. Additionally or alternatively, the device 100 can be used in detecting at least one predetermined condition from skin selected from temperature, moisture, ionic content and/or dissolution rate of at least one chemical substance released from the functional layer 20, for example.

The solution disclosed hereby allows for producing smart-body on-skin ALD-wound care products for different types of wounds. By using ALD, controlled release of desired drugs can be realized to control infection rates and to promote wound repair. By using ALD, gas-fluid exchange between the wound and the ambient can be modulated by implementing precision control over the size of (micro)pores to maintain the wound homeostasis.

## Claims

1. A device for wound care (100), comprising:
a base layer (10);
a functional layer (20) supported on the base layer and containing at least one chemical substance (21) suitable for wound care; and
a coating film (22) laid over said functional layer and having a skin-contact surface (22A),
wherein said coating film (22) is configured, upon being brought into contact with skin, via the skin-contact surface (22A), to gradually dissolve and to release said chemical substance (21) to skin through the skin-contact surface in a time-controlled manner, and
wherein the coating film (22) is an atomic layer deposition (ALD) film.

2. The device (100) of claim 1, configured as a bandage, a patch, a tape, a roll, a plaster, and a wound dressing.

3. The device (100) of any one of claims 1 and 2, wherein the coating film (22) comprises at least one compound selected from aluminium oxide, titanium oxide, silicon oxide, and a combination thereof.

4. The device (100) of any preceding claim, in which the functional layer comprises at least one chemical substance with antimicrobial- and/or antibacterial activity.

5. The device (100) of any preceding claim, further comprising at least one sensor (30, 40) and related circuitry for detecting, from skin, of at least one predetermined condition selected from temperature, moisture, ionic content and/or dissolution rate of chemical substance(s).

6. The device (100) of any preceding claim, comprising a number of the functional layers (20) arranged into a stack structure and alternating, in said stack structure, by means of a corresponding number of the coating films (22), wherein each functional layer contains the same chemical substance or different chemical substances.

7. The device (100) of claim 1, wherein the coating film (22) is configured to enable adjustment of a chemical substance release gradient over a dissolving time of said chemically deposited film coating.

8. A method for manufacturing a wound care device (100), comprising:
- obtaining a base layer (10) and applying a functional layer (20) containing at least one chemical substance (21) suitable for wound care onto said base layer,
- depositing, by atomic layer deposition (ALD), a coating film (22) with a skin-contact surface (22A) over said functional layer (20) such, that upon bringing said coating film into contact with skin, via the skin-contact surface (22A), the coating film gradually dissolves and releases said chemical substance (21) to skin through the skin-contact surface in a time-controlled manner.

9. The method of claim 8, wherein the coating film comprises at least one metal oxide.

10. The method of any one of claims 8 and 9, wherein the coating film (22) comprises at least one compound selected from aluminium oxide, titanium oxide, silicon oxide, or a combination thereof.

11. The method of any one of claims 8-10, further comprising a step of arranging, within the device (100), at least one sensor (30, 40) and related circuitry for detecting, from skin, of at least one predetermined condition selected from temperature, moisture, ionic content and/or dissolution rate of chemical substance(s).

12. The device as defined in any one of claims 1-7 for use in a method of wound care comprising detecting at least one predetermined condition from skin selected from temperature, moisture, ionic content and/or dissolution rate of chemical substances, wherein said at least one predetermined condition is detected using at least one sensor (30, 40) and external measurement equipment that receives signals from said sensors.

## Patentansprüche

1. Wundpflegevorrichtung (100), umfassend:
eine Basisschicht (10);
eine Funktionsschicht (20), die auf der Basisschicht aufliegt und mindestens eine chemische Substanz (21), die sich zur Wundpflege eignet, enthält; und
einen Beschichtungsfilm (22), der die Funktionsschicht bedeckt und eine Hautkontaktfläche (22A) aufweist,
wobei der Beschichtungsfilm (22) dazu ausgelegt ist, sich bei Hautkontakt über die Hautkontaktfläche (22A) langsam aufzulösen und die chemische Substanz (21) auf zeitgesteuerte Weise durch die Hautkontaktfläche an die Haut freizusetzen, und
wobei der Beschichtungsfilm (22) ein Atomlagenabscheidungsfilm (ALD-Film) ist.

2. Vorrichtung (100) nach Anspruch 1, die als ein Verband, ein Pflaster, ein Band, eine Rolle, ein Wundschnellverband und eine Wundauflage ausgelegt ist.

3. Vorrichtung (100) nach einem der Ansprüche 1 und 2, wobei der Beschichtungsfilm (22) mindestens eine Verbindung, die aus Aluminiumoxidoxid, Titanoxid, Siliziumoxid und einer Kombination davon ausgewählt ist, umfasst.

4. Vorrichtung (100) nach einem der vorhergehenden Ansprüche, wobei die Funktionsschicht mindestens eine chemische Substanz mit antimikrobieller und/oder antibakterieller Aktivität umfasst.

5. Vorrichtung (100) nach einem der vorhergehenden Ansprüche, ferner umfassend mindestens einen Sensor (30, 40) und eine zugehörige Schaltlogik zum Erkennen an der Haut von mindestens einer vorbestimmten Bedingung, ausgewählt aus Temperatur, Feuchtigkeit, Ionengehalt und/oder Auflösungsgeschwindigkeit der chemischen Substanz(en).

6. Vorrichtung (100) nach einem der vorhergehenden Ansprüche, umfassend eine Anzahl von Funktionsschichten (20), die in einer Stapelstruktur angeordnet sind und sich in der Stapelstruktur mit einer entsprechenden Anzahl der Beschichtungsfilme (22) abwechseln, wobei jede Funktionsschicht dieselbe chemische Substanz oder unterschiedliche chemische Substanzen enthält.

7. Vorrichtung (100) nach Anspruch 1, wobei der Beschichtungsfilm (22) dazu ausgelegt ist, die Anpassung eines Freisetzungsgradienten einer chemischen Substanz über eine Auflösungsdauer der chemisch abgeschiedenen Filmbeschichtung zu ermöglichen.

8. Verfahren zum Herstellen einer Wundpflegevorrichtung (100), umfassend:
- Erhalten einer Basisschicht (10) und Auftragen einer Funktionsschicht (20), die mindestens eine chemische Substanz (21), die sich zur Wundpflege eignet, enthält, auf die Basisschicht,
- Abscheiden eines Beschichtungsfilms (22) mit einer Hautkontaktfläche (22A) über der Funktionsschicht (20) mittels Atomlagenabscheidung (ALD), sodass sich der Beschichtungsfilm beim Hautkontakt des Beschichtungsfilms durch die Hautkontaktfläche (22A) langsam auflöst und die chemische Substanz (21) auf zeitgesteuerte Weise durch die Hautkontaktfläche an die Haut freisetzt.

9. Verfahren nach Anspruch 8, wobei der Beschichtungsfilm mindestens ein Metalloxid umfasst.

10. Verfahren nach einem der Ansprüche 8 und 9, wobei der Beschichtungsfilm (22) mindestens eine Verbindung, die aus Aluminiumoxidoxid, Titanoxid, Siliziumoxid oder einer Kombination davon ausgewählt ist, umfasst.

11. Verfahren nach einem der Ansprüche 8 bis 10, ferner umfassend einen Schritt des Anordnens von mindestens einem Sensor (30, 40) und einer zugehörigen Schaltlogik zum Erkennen an der Haut von mindestens einer vorbestimmten Bedingung, ausgewählt aus Temperatur, Feuchtigkeit, Ionengehalt und/oder Auflösungsgeschwindigkeit der chemischen Substanz(en), in der Vorrichtung (100).

12. Vorrichtung, wie in einem der Ansprüche 1 bis 7 definiert, zur Verwendung in einem Wundpflegeverfahren, umfassend das Erkennen an der Haut von mindestens einer vorbestimmten Bedingung, ausgewählt aus Temperatur, Feuchtigkeit, Ionengehalt und/oder Auflösungsgeschwindigkeit von chemischen Substanzen, wobei die mindestens eine vorbestimmte Bedingung mit mindestens einem Sensor (30, 40) und einer externen Messvorrichtung, die Signale von den Sensoren empfängt, erkannt wird.

## Revendications

1. Dispositif pour traitement des plaies (100), comprenant :
une couche de base (10) ;
une couche fonctionnelle (20) supportée sur la couche de base et contenant au moins une substance chimique (21) appropriée pour le traitement des plaies ; et
un film de revêtement (22) posé sur ladite couche fonctionnelle et ayant une surface de contact avec la peau (22A),
ledit film de revêtement (22) étant configuré, après avoir été mis en contact avec la peau, via la surface de contact avec la peau (22A), pour se dissoudre graduellement et pour libérer ladite substance chimique (21) sur la peau par la surface de contact avec la peau de manière contrôlée dans le temps et
le film de revêtement (22) étant un film par dépôt de couche atomique (ALD).

2. Dispositif (100) selon la revendication 1, configuré comme un bandage, un patch, une bande, un rouleau, un plastique et un pansement de blessure.

3. Dispositif (100) selon l'une quelconque des revendications 1 et 2, dans lequel le film de revêtement (22) comprend au moins un composé choisi parmi l'oxyde d'aluminium, l'oxyde de titane, l'oxyde de silicium et une de leurs combinaisons.

4. Dispositif (100) selon l'une quelconque des revendications précédentes, dans lequel la couche fonctionnelle comprend au moins une substance chimique avec une activité anti microbienne et/ou anti bactérienne.

5. Dispositif (100) selon l'une quelconque des revendications précédentes, comprenant en outre un capteur (30, 40) et le circuit associé pour la détection, à partir de la peau, d'au moins une condition prédéterminée choisie parmi la température, l'humidité, la teneur ionique et/ou le taux de dissolution de (des) la substance(s) chimique(s).

6. Dispositif (100) selon l'une quelconque des revendications précédentes, comprenant un nombre des couches fonctionnelles (20) disposées dans une structure en pile et en alternance, dans ladite structure en pile, au moyen d'un nombre correspondant des films de revêtement (22), chaque couche fonctionnelle contenant la même substance chimique ou des substances chimiques différentes.

7. Dispositif (100) selon la revendication 1, dans lequel le film de revêtement (22) est configuré pour permettre l'ajustement d'un gradient de libération de substance chimique sur un temps de dissolution dudit film de revêtement déposé chimiquement.

8. Procédé de fabrication d'un dispositif de traitement de plaies (100), comprenant :
- l'obtention d'une couche de base (10) et l'application d'une couche fonctionnelle (20) contenant au moins une substance chimique (21) appropriée pour le traitement des plaies sur ladite couche de base,
- le dépôt, par dépôt de couche atomique (ALD), d'un film de revêtement (22) avec une surface de contact avec la peau (22A) sur ladite couche fonctionnelle (20) telle, qu'après avoir mis ledit film de revêtement en contact avec la peau, via la surface de contact avec la peau (22A), le film de revêtement se dissout graduellement et libère ladite substance chimique (21) sur la peau par la surface de contact avec la peau de manière contrôlée dans le temps.

9. Procédé selon la revendication 8, dans lequel le film de revêtement comprend au moins un oxyde métallique.

10. Procédé selon l'une quelconque des revendications 8 et 9, dans lequel le film de revêtement (22) comprend au moins un composé choisi parmi l'oxyde d'aluminium, l'oxyde de titane, l'oxyde de silicium ou une de leurs combinaisons.

11. Procédé selon l'une quelconque des revendications 8 à 10, comprenant en outre une étape de disposition, dans le dispositif (100), d'au moins un capteur (30, 40) et du circuit associé pour la détection, à partir de la peau, d'au moins une condition prédéterminée choisie parmi la température, l'humidité, la teneur ionique et/ou le taux de dissolution de (des) la substance(s) chimique(s) .

12. Dispositif tel que défini selon l'une quelconque des revendications 1 à 7 destiné à être utilisé dans un procédé de traitement des plaies comprenant la détection d'au moins une condition prédéterminée à partir de la peau choisie parmi la température, l'humidité, le teneur ionique et/ou le taux de dissolution de substance chimiques, ladite au moins une condition prédéterminée étant détectée en utilisant au moins un capteur (30, 40) et un équipement de mesure externe qui reçoit les signaux desdits capteurs.
